Europäisches Patentamt

European Patent Office

Office européen des brevets

(19)

(11) Publication number: **0 419 091 A1**

# EUROPEAN PATENT APPLICATION

(21) Application number: **90309700.4**

(22) Date of filing: **05.09.90**

(51) Int. Cl.⁵: **C12N 15/12, C07K 13/00, C12P 21/02**

(30) Priority: **07.09.89 US 404179**
**20.03.90 US 496449**

(43) Date of publication of application:
**27.03.91 Bulletin 91/13**

(84) Designated Contracting States:
**GR**

(71) Applicant: **SCHERING CORPORATION**
**2000 Galloping Hill Road**
**Kenilworth New Jersey 07033(US)**

(72) Inventor: **Galizzi, Jean-Pierre**
**27 chemin des peupliers BP11**
**F-69572 Dardilly(FR)**
Inventor: **Harada, Nobuyuki**
**2310 St. Francis Drive**
**Palo Alto, California 94303(US)**
Inventor: **Miyajima, Atsushi**
**4159 Drake Avenue**
**Palo Alto, California 94306(US)**

(74) Representative: **Ritter, Stephen David et al**
**Mathys & Squire 10 Fleet Street**
**London EC4Y 1AY(GB)**

(54) **Interleukin-4 receptors.**

(57) Nucleic acids are provided which encode mammalian interleukin-4 receptors, and which serve as probes for related receptors by cross hybridization. Soluble forms of human interleukin-4 receptors are useful as antagonists of interleukin-4 in the treatment of conditions associated with excessive IgE production.

EP 0 419 091 A1

## INTERLEUKIN-4 RECEPTORS

### Field of the Invention

The invention relates generally to the mammalian IL-4 (interleukin-4) receptor, and more particularly to the synthesis of the IL-4 receptor by recombinant cells and to the manufacture and use of such receptors for screening agonists and antagonists and for reducing in vivo levels of IL-4 in certain disease conditions.

### BACKGROUND

Several forms of mammalian IL-4 have been cloned and characterized: e.g. Lee et al.; Proc. Natl. Acad. Sci. USA , Vol. 83, pgs. 2061-2065 (1986); and Yokota et al., Proc. Natl. Acad. Sci. USA , Vol. 83, pgs. 5894-5898 (1986). More recently, studies have shown that IL-4 may play an important role in hypersensitivity reactions, particularly in those due to immunoglobulin E (IgE): e.g. Coffman et al., J. Immunol. , Vol. 4538-4541 (1986); Finkelman et al., Proc. Natl. Acad. Sci. USA , Vol. 83, pgs. 9675-9678 (1986); Snapper et al., Science , Vol. 236, pgs. 944-947 (1987); and Mosmann et al., Ann. Rev. Immunol. , Vol. 7, pgs. 145-173 (1989). In particular, Finkelman et al. (cited above) have shown that IgE responses can be suppressed by the administration of antibodies capable of blocking the biological activity of IL-4, thereby suggesting an alternative route for treating allergic conditions characterized by excessive IgE production.

Currently, glucocorticoid steroids are the most effective drugs for treating such disorders. However, prolonged steroid treatment is associated with many deleterious side effects (Goodman and Gillman, The Pharmacological Basis of Therapeutics , 6th Ed. (MacMillan, New York, 1980)). Accordingly, the availability of alternative approaches to the treatment of immune disorders associated with excessive IgE production could have important clinical utility.

### SUMMARY OF THE INVENTION

The invention is directed to mammalian IL-4 receptors ("IL-4Rs") in soluble and in membrane-bound forms, nucleic acids encoding the same, and the use of soluble forms of the receptors ("sIL-4Rs") as antagonists of IL-4 to treat conditions characterized by excessive IgE production. The invention also includes the nucleic acids (1) that are effectively homologous to the murine nucleotide sequence defined by Formula I below, and (2) that encode a polypeptide capable of binding IL-4 derived from the same species as the nucleic acid. Preferably, the soluble IL-4Rs bind IL-4 with substantially the same affinity as the native membrane-bound IL-4Rs, or with even greater affinity.

```
        5'-      GCGCGGCGTG    GAGCCTGAAC    TCGCAGGTTC
   TGGCTGGACT    TCTCGAAGCT    GAGGAGAAGC    AGAGGGACCT
   GGCTTCTGAT    TTTGGATCTG    CGTGCTTGCT    GGTTCTGGCG
   CCTGCTGGTC    TTCTTCCTGT    AACCTAGGAC    TCGGGGCTTG
   CACATGCTTT    TTTTTTGAAG    TTGCTGGAGA    GGGAGCCCAG
   GACCTTGTGC    AGGCACCTTT    TGTGTCCCCA    ATGGGGCGGC
   TTTGCACCAA    GTTCCTGACC    TCTGTGGGCT    GTCTGATTTT
   GCTGTTGGTG    ACTGGATCTG    GGAGCATCAA    GGTCCTGGGT
   GAGCCCACCT    GCTTCTCTGA    CTACATCCGC    ACTTCCACGT
   GTGAGTGGTT    CCTGGATAGC    GCTGTGGACT    GCAGTTCTCA
   GCTCTGCCTA    CACTACAGGC    TGATGTTCTT    CGAGTTCTCT
   GAAAACCTCA    CATGCATCCC    GAGGAACAGT    GCCAGCACTG
   TGTGTGTGTG    CCACATGGAA    ATGAATAGGC    CGGTCCAATC
   AGACAGATAC    CAGATGGAAC    TGTGGGCTGA    GCACAGACAG
   CTGTGGCAGG    GCTCCTTCAG    CCCCAGTGGT    AATGTGAAGC
   CCCTAGCTGG    AGACAACCTC    ACACTCCACA    CCAATGTGTC
   CGACGAATGG    CTGCTGACCT    GGAATAACCT    GTACCCATCG
```

```
AACAACTTAC    TGTACAAAGA    CCTCATCTCC    ATGGTCAACA
TCTCCAGAGA    GGACAACCCT    GCAGAATTCA    TAGTCTATAA
TGTGACCTAC    AAGGAACCCA    GGCTGAGCTT    CCCGATCAAC
ATCCTGATGT    CAGGGGTCTA    CTATACGGCG    CGTGTGAGGG
TCAGATCCCA    GATACTCACT    GGCACCTGGA    GTGAGTGGAG
TCCTAGCATC    ACGTGGTACA    ACCACTTCCA    GCTGCCCCTG
ATACAGCGCC    TTCCACTGGG    GGTCACCATC    TCCTGCCTCT
GCATCCCGTT    GTTTTGCCTG    TTCTGTTACT    TCAGCATTAC
CAAGATTAAG    AAGATATGGT    GGGACCAGAT    TCCCACCCCA
GCACGCAGTC    CCTTGGTGGC    CATCATCATT    CAGGATGCAC
AGGTGCCCCT    CTGGGATAAG    CAGACCCGAA    GCCAGGAGTC
AACCAAGTAC    CCGCACTGGA    AAACTTGTCT    AGACAAGCTG
CTGCCTTGCT    TGCTGAAGCA    CAGAGTAAAG    AAGAAGACAG
ACTTCCCGAA    GGCTGCCCCA    ACCAAGTCTC    TCCAGAGTCC
TGGAAAGGCA    GGCTGGTGTC    CCATGGAGGT    CAGCAGGACC
GTCCTCTGGC    CAGAGAATGT    TAGTGTCAGT    GTGGTGCGCT
GTATGGAGCT    GTTTGAGGCC    CCAGTACAGA    ATGTGGAGGA
GGAAGAAGAT    GAGATAGTCA    AAGAGGACCT    GAGCATGTCA
CCTGAGAACA    GCGGAGGCTG    CGGCTTCCAG    GAGAGCCAGG
CAGACATCAT    GGCTCGGCTC    ACTGAGAACC    TGTTTTCCGA
CTTGTTGGAG    GCTGAGAATG    GGGGCCTTGG    CCAGTCAGCC
TTGGCAGAGT    CATGCTCCCC    TCTGCCTTCA    GGAAGTGGGC
AGGCTTCTGT    ATCCTGGGCC    TGCCTCCCCA    TGGGGCCCAG
TGAGGAGGCC    ACATGCCAGG    TCACAGAGCA    GCCTTCACAC
CCAGGCCCTC    TTTCAGGCAG    CCCAGCCCAG    AGTGCACCTA
CTCTGGCTTG    CACGCAGGTC    CCACTTGTCC    TTGCAGACAA
TCCTGCCTAC    CGGAGTTTTA    GTGACTGCTG    TAGCCCGGCC
CCAAATCCTG    GAGAGCTGGC    TCCAGAGCAG    CAGCAGGCTG
ATCATCTGGA    AGAAGAGGAG    CCTCCAAGCC    CGGCTGACCC
CCATTCTTCA    GGGCCTCCAA    TGCAGCCAGT    GGAGAGCTGG
GAGCAGATCC    TTCACATGAG    TGTCCTGCAG    CATGGGGCAG
CTGCTGGCC    -3'
```

<u>Formula I</u>

In Formula I, A, C, G, and T represent deoxyadenosine, deoxycytidine, deoxyguanosine, and thymidine, respectively. Standard notation is used below to designate L-amino acids: e.g. Cohn, <u>Meth. Enzymol.</u>, Vol. 106, pg. 4 (1984).

Brief Description of the Drawings

Figure 1 illustrates data describing the ability of mouse sIL-4R to block the binding of IL-4 to HT-2 cells.

Figure 2 illustrates data describing the ability of mouse sIL-4R to inhibit the biological activity of IL-4.

Figure 3 is a restriction map of the vector pME18S.

Figures 4A and 4B illustrate the binding of $^{125}$I-human IL-4 to TF1 cells (A) and to COS 7 cells (B), where both types of cells are transiently transfected with pME18S-hIL-4R.

Figure 5 (in two parts A and B on two sheets) is a comparison of the amino acid sequences of human IL-4R (upper line of each pair) and mouse IL-4R (lower line of each pair), and shows the leader sequence of human IL-4R singly underlined and the transmembrane domain of human IL-4R doubly underlined.

## DETAILED DESCRIPTION OF THE INVENTION

### I. Obtaining and Expressing IL-4R cDNAs

The term "effectively homologous" as used herein means that the nucleotide sequence is capable of being detected by a hybridization probe derived from a cDNA clone of the invention. The exact numerical measure of homology necessary to detect nucleic acids coding for an IL-4R depends on several factors including (1) the homology of the probe to sequences that do not encode IL-4R but are associated with the target nucleic acids, (2) the stringency of the hybridization conditions, (3) whether single-stranded or double-stranded probes are employed, (4) whether RNA or DNA probes are employed, (5) the measures taken to reduce nonspecific binding of the probe, (6) the nature of the method used to label the probe, (7) the fraction of guanidine and cytosine bases in the probe, (8) the distribution of mismatches between probe and target, (9) the size of the probe, and the like. Preferably, an effectively homologous nucleic acid sequence is at least seventy percent (70%) homologous to the cDNA of the invention. More preferably, an effectively homologous nucleic acid is at least ninety percent (90%) homologous to the cDNA of the invention. Most particularly, an effectively homologous nucleic acid sequence is one whose cDNA can be isolated by a probe based on the nucleic acid sequence of Formula I using the hybridization protocol described in the Examples with no more than a few false positive signals, e.g. fewer than a hundred. There is an extensive literature that provides guidance in selecting conditions for such hybridizations: e.g. Hames et al., Nucleic Acid Hybridization: A Practical Approach (IRL Press, Washington, D.C., 1985); Gray et al., Proc. Natl. Acad. Sci. USA , Vol. 80, pgs. 5842-5846 (1983); Kafatos et al., Nucleic Acids Research , Vol. 7, pgs. 1541-1552 (1979); and Williams, Genetic Engineering , Vol. 1, pgs. 1-59 (1981). By way of example, the nucleic acid of Formula I can be used as a probe in colony hybridization assays as described by Benton and Davis in Science , Vol. 196, pg. 180 (1977). Preferably, low stringency conditions are employed for the probe employed; the dissociation temperature depends on probe length, and increases as the probe length increases. For example, for a probe of about 20-40 bases a typical protocol including the steps of: prehybridizing, hybridizing, and washing; is as follows: (1) prehybridizing: incubate nitrocellulose filters containing the denatured target DNA for 3-4 hours at 55°C in 5x Denhardt's solution, 5x SSPE (20x SSPE consists of 174 g NaCl, 27.6 g $NaH_2PO_4.H_2O$, and 7.4 g EDTA in 800 ml water adjusted to pH 7.4 with 10 N NaOH), 0.1% SDS, and 100 μg/ml denatured salmon sperm DNA, (2) hybridizing: incubate filters in prehybridization solution plus probe at 55°C for 2 hours, (3) washing: three 15-minute washes in 300-500 ml volumes of 6x SSC and 0.1% SDS at room temperature, followed by a final 1-1.5-minute wash in 300-500 ml of 1x SSC and 0.1% SDS at 55°C. Other equivalent procedures are well known in the art.

Homology as the term is used herein is a measure of similarity between two nucleotide (or amino acid) sequences. Homology is expressed as the fraction or percentage of matching bases (or amino acids) after two sequences (possibly of unequal length) have been aligned. The term alignment is used in the sense defined by Sankoff and Kruskal in Chapter 1 of "Time Warps, String Edits, and Macromolecules: The Theory and Practice of Sequence Comparison" (Addison-Wesley, Reading, MA, 1983). Roughly, two sequences are aligned by maximizing the number of matching bases (or amino acids) between the two sequences with the insertion of a minimal number of "blank" or "null" bases into either sequence to bring about the maximum overlap. Given two sequences, algorithms are available for computing their homology, e.g. Needleham and Wunsch, J. Mol. Biol. , Vol. 48, pgs. 443-453 (1970), and Sankoff and Kruskal (cited above) pgs. 23-29. Also, commercial services and software packages are available for performing such comparisons, e.g. Intelligenetics, Inc. (Mountain View, CA), and University of Wisconsin Genetics Computer Group (Madison, Wisconsin).

Probes based on the nucleic acid sequence of Formula I can be synthesized on commercially available DNA synthesizers, e.g. Applied Biosystems model 381A, using standard techniques, e.g. Gait, Oligonucleotide Synthesis: A Practical Approach, (IRL Press, Washington D.C., 1984). Preferably the probe is at least 18-30 bases long, more preferably at least 100-200 bases long. Probes of the invention can be labeled in a variety of ways standard in the art; e.g. with radioactive labels (see e.g. Berent et al., Biotechniques , pgs. 208-220 (May/June 1985), Meinkoth et al., Anal. Biochem. , Vol. 138, pgs. 267-284 (1984), and Szostak et al., Meth. Enzymol. , Vol. 68, pgs. 419-429 (1979)), or with non-radioactive labels (see e.g. Chu et al., DNA , Vol. 4, pgs. 327-331 (1985), Jablonski et al., Nucleic Acids Research , Vol. 14, pgs. 6115-6128 (1986)).

Hybridization probes can also be used to screen candidate sources of IL-4R mRNA prior to library construction: e.g. by RNA blotting, Maniatis et al., Molecular Cloning: A Laboratory Manual, pgs. 202-203 (Cold Spring Harbor Laboratory, N.Y., 1982); or Hames and Higgins, eds., pgs. 139-143 in Nucleic Acids Hybridization (IRL Press, Washington, D.C., 1985). Sources of mRNA encoding the desired polypeptides include cell populations or cell lines that express, or can be induced to express, large numbers of IL-4 receptors on their surfaces, e.g. in excess of 3000-5000. Such cells include T cells, T cell lines, mast cell lines, and the like. In general, suitable T cells can be obtained from a variety of sources, such as mammalian (e.g. human) spleen, tonsils and peripheral blood. Preferably, mRNA is obtained from T cells extracted from the peripheral blood and treated with IL-4 to induce an increased expression of IL-4R. Such induction can be accomplished by first exposing the human T cells to 1 $\mu$g/ml of phytohemaglutinin (PHA) for about 3 days, followed by exposure to about 1000 picomolar IL-4. After about 24 hours, the cells express a 2-3 fold increase in IL-4Rs.

If the mRNAs encoding a desired IL-4R make up a very small fraction of the total mRNA, it may be necessary to enrich the fractional concentration in order to make practical the screening procedure for detecting cDNA clones of interest. Such procedures are standard in the art and are disclosed in several papers and references, such as: Maniatis et al., pgs. 225-228, cited above; Suggs et al., Proc. Natl. Acad. Sci. USA , Vol. 78, pgs. 6613-6617 (1981); Parnes et al., Proc. Natl. Acad. Sci. USA , Vol. 78, pgs. 2253-2257 (1981); Davis et al., Proc. Natl. Acad. Sci. USA , Vol. 81, pgs. 2194-2198 (1984); or the like.

Once a cDNA of the invention has been cloned, a wide range of expression systems (i.e. host-expression vector combinations) can be used to produce the proteins of the invention. Possible types of host cells include, but are not limited to, bacterial, yeast, insect, mammalian, and the like. Many reviews are available which provide guidance for making choices and/or modifications of specific expression systems: e.g. de Boer and Shepard, "Strategies for Optimizing Foreign Gene Expression in Escherichia coli," pgs. 205-247 in Kroon, ed. Genes: Structure and Expression (John Wiley & Sons, New York, 1983), review several E. coli expression systems; Kucherlapati et al., Critical Reviews in Biochemistry , Vol. 16, Issue 4, pgs. 349-379 (1984); and Banerji et al., Genetic Engineering , Vol. 5, pgs. 19-31 (1983) review methods for transfecting and transforming mammalian cells; Reznikoff and Gold, eds., Maximizing Gene Expression (Butterworths, Boston, 1986) review selected topics in gene expression in E. coli , yeast, and mammalian cells; and Thilly, Mammalian Cell Technology (Butterworths, Boston, 1986) reviews mammalian expression systems (to mention a few). Likewise, many reviews are available which describe techniques and conditions for linking and/or manipulating specific cDNAS and expression control sequences to create and/or modify expression vectors suitable for use with the present invention and to create and modify cDNAS to produce soluble forms of the receptor: e.g. Maniatis et al., Molecular Cloning: A Laboratory Manual (Cold Spring Harbor Laboratory, N.Y., 1982); Glover, DNA Cloning: A Practical Approach, Vol. I and II (IRL Press, Oxford, 1985); and Perbal, A Practical Guide to Molecular Cloning (John Wiley & 50ns, N.Y., 1984), to name only a few.

An E. coli expression system is disclosed by Riggs in U.S. Patent 4,431,739, which is incorporated by reference. A particularly useful prokaryotic promoter for high expression in E. coli is the tac promoter, disclosed by de Boer in U.S. Patent 4,551,433, which is incorporated herein by reference. Secretion expression vectors are also available for E. coli hosts. Particularly useful are the pIN-III-ompA vectors, disclosed by Ghrayeb et al. in EMBO J. , Vol. 3, pgs. 2437-2442 (1984), in which the cDNA to be transcribed is fused to the portion of the E. coli OmpA gene encoding the signal peptide of the ompA protein which, in turn, causes the mature protein to be secreted into the periplasmic space of the bacteria. U.S. Patents 4,336,336 and 4,338,397 also disclose secretion expression vectors for prokaryotes and accordingly are incorporated by reference. Numerous strains of bacteria are suitable hosts for prokaryotic expression vectors including strains of E. coli , such as W3110 (ATCC No. 27325), JA221, C600, ED767, DH1, LE392, HB101, X1776 (ATCC No. 31244), X2282, RR1 (ATCC No. 31343) MRCI; strains of Bacillus subtilis ; and other enterobac teriaceae such as Salmonella typhimurium or Serratia marcescens , and various species of Pseudomonas . General methods for deriving bacterial strains, such as E. coli K12 X1776, useful in the expression of eukaryotic proteins are disclosed by Curtis III in U.S. Patent 4,190,495, which is accordingly incorporated by reference.

In addition to prokaryotic and eukaryotic microorganisms, expression systems comprising cells derived from multicellular organisms may also be used to produce proteins of the invention. Of particular interest are mammalian expression systems because their posttranslational processing machinery is more likely to produce biologically active mammalian proteins. Several DNA tumor viruses have been used as vectors for mammalian hosts. Particularly important are the numerous vectors which comprise SV40 replication, transcription, and/or translation control sequences coupled to bacterial replication control sequences: e.g. the pcD vectors developed by Okayama and Berg, disclosed in Mol. Cell. Biol. , Vol. 2, pgs. 161-170(1982) and Mol. Cell. Biol. , Vol. 3, pgs. 280-289 (1983), both of which are incorporated herein by reference; the SV40 vectors disclosed by Hamer in Genetic Engineering , Vol. 2, pgs. 83-100 (1980), and U.S. Patent 4,599,308, both of which are incorporated herein by reference; and the vectors additionally containing adenovirus regulatory elements, disclosed by Kaufman and Sharp, in Mol. Cell. Biol. , Vol. 2, pgs. 1304-1319 (1982), and Clark et al., in U.S. patent 4,675,285, both of which are incorporated herein by reference. COS7 monkey cells, described by Gluzman, Cell , Vol. 23, pgs. 175-182 (1981) and available from the ATCC (accession no. CRL 1651), are usually the preferred hosts for the above vectors.

Preferably, soluble forms of the IL-4Rs are produced by introducing a stop codon prior to (i.e. in the 5'- or "upstream" direction of) the coding region for the transmembrane and intracellular portions of the IL-4R cDNA. This is conveniently done by site-specific mutagenesis, e.g. as taught by Zoller and Smith, Meth. Enzymol. , Vol. 100, pgs. 468-500 (1983), and the like. Extracellular, transmembrane, and intracellular domains of receptor cDNAs are readily identified by the hydrophobicity of the encoded amino acids in the largest open reading frame. For example, the transmembrane domain is a segment containing from about 20-25 residues having high average hydrophobicity, e.g. Wickner, Science , Vol. 210, pgs. 861-868 (1980), and Greene et al., Ann. Rev. Immunol. , Vol. 4, pgs. 69-95 (1986). The amino acids in the N-terminal direction from the transmembrane domain constitute the extracellular domain, and the amino acids in the C-terminal direction from the transmembrane domain constitute the intracellular domain.

## II. Soluble Interleukin-4 Receptor Binding Assays

The affinity, or strength of binding, of soluble IL-4Rs to IL-4 is measured in relation to the binding of IL-4 to a standardized number of membrane-bound IL-4Rs. That is, the affinity of a soluble IL-4R for IL-4 is determined in a competition assay with membrane-bound IL-4R. The membrane-bound IL-4Rs can be the natural IL-4Rs of a cell line responsive to IL-4, e.g. a helper T cell line. Affinity can be measured in terms of inhibition of biological activity or the inhibition of binding of $^{125}$I-labeled IL-4 to the membrane-bound IL-4Rs.

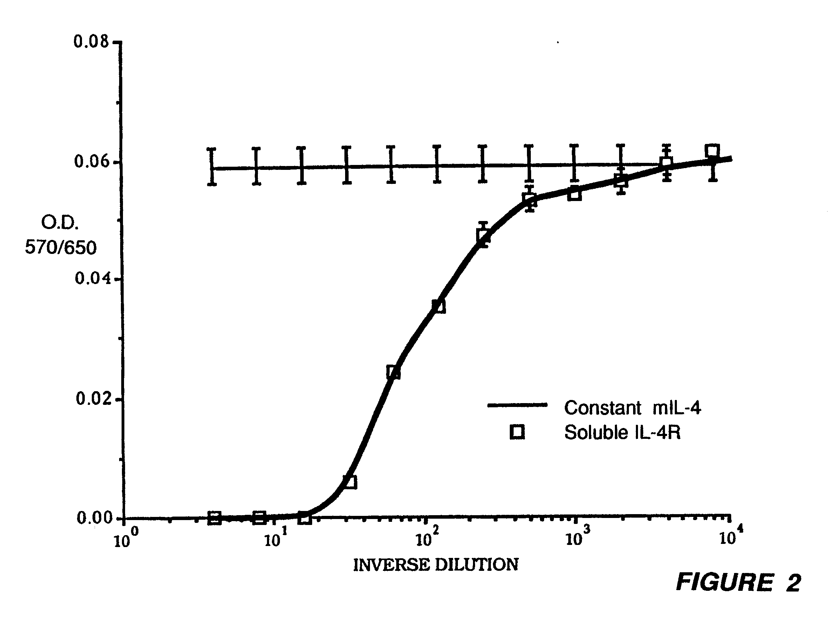

Typically the biological activity measured is cell growth, as determined by tritiated thymidine incorporation, or like assays of cell growth, e.g. Mosmann, J. Immunol. Meth. , Vol. 65, pgs. 55-63 (1983). Thus, inhibition of IL-4-induced cell growth is measured by exposing each of a series of cell cultures to a constant initial concentration of IL-4 and a different initial concentration of soluble IL-4R, and then examining the relationship of soluble IL-4R concentration to cell growth., e.g. as illustrated by Figure 2. Preferably, a cell-line responsive to IL-4 is used, but T cells from peripheral blood lymphocytes can also be used. Preferably, the concentration of IL-4 is at or near the level where the biological response becomes saturated, i.e. the level where additional IL-4 has no measurable effect on the cells. Accordingly, to apply the inhibition assay for particular cell types may require some routine experimentation to establish the saturation concentration. By way of example, inhibition of human T cell growth factor activity can be assayed by the following steps: (1) aliquots of washed human peripheral blood lymphocytes (about $2 \times 10^5$ in 50 $\mu$l) previously stimulated with phytohemagglutinin (PHA) for 7 days and subsequently cultured for 7 days with IL-2 are added to microtiter plates each containing a known concentration of IL-4, (2) dilutions (e.g. in 50 $\mu$l) of the sIL-4R are added to each well, (3) the cells are incubated for 72 hours at 37°C, (4) tritiated thymidine (about 20 ml at 20 $\mu$Ci/ml) is added to each well, and (5) cells are harvested onto filter paper, washed, and counted in a scintillation counter.

For the alternative assay, IL-4 is iodinated by standard commercially available techniques, e.g. reaction with aqueous Na$^{125}$I and with 1,3,4,6-tetrachloro-3$\alpha$,6$\alpha$-diphenylglycoluril (described by Fraker et a]., Biochem Biophys. Res. Commun. , Vol. 80, pgs. 849-857 (1978) and available from Pierce Chemical Co. as IODO-GEN). Generally, the binding assay is as described by Lowenthal et al., J. Immunol. , Vol. 140, pgs. 456-464 (1988), which is incorporated by reference. Human cell lines having IL-4R suitable for use with the binding assays include Jiyoye cells and Jurkat cells, the former being available from the American Type Culture Collection under accession number CCL 87.

III. Purification and Pharmaceutical Compositions

When polypeptides of the present invention are expressed in soluble form, for example as a secreted product of transformed yeast or mammalian cells, they can be purified according to standard procedures of the art, including steps of ammonium sulfate precipitation, ion exchange chromatography, gel filtration, electrophoresis, affinity chromatography, and/or the like; e.g. "Enzyme Purification and Related Techniques," Methods in Enzymology , 22:233-577 (1977), and Scopes, R., Protein Purification: Principles and Practice, Springer-Verlag, New York [1982]) provide guidance in such purifications. Likewise, when polypeptides of the invention are expressed in insoluble form, for example as aggregates, inclusion bodies or the like, they can be purified by standard procedures in the art, including separating the inclusion bodies from disrupted host cells by centrifugation, solubilizing the inclusion bodies with chaotropic agents and reducing agents, diluting the solubilized mixture, and lowering the concentration of chaotropic agent and reducing agent so that the polypeptide takes on a biologically active conformation. The latter procedures are disclosed in the following references, which are incorporated by reference: Winkler et al., Biochemistry , 25: 4041-4045 (1986); Winkler et al., Biotechnology , 3: 992-998 (1985); Koths et al., U.S. patent 4,569,790; and European patent applications 86306917.5 and 86306353.3.

For preparing pharmaceutical compositions containing the polypeptides described by this invention, these polypeptides are compounded by admixture with preferably inert, pharmaceutically acceptable carriers. Suitable carriers and processes for their preparation are well known in the art, e.g., Remington's Pharmaceutical Sciences and U.S. Pharmacopeia: National Formulary (Mack Publishing Company, Easton, PA,1984). The preferred course of administration is parenteral and can include use of an implantable drug delivery system, e.g. Urquhart et al., Ann. Rev. Pharmacol. Toxicol. , Vol. 24, pgs. 199-236 (1984).

As used herein "effective amount" means an amount sufficient to ameliorate a symptom of an autoimmune condition. The effective amount for a particular patient may vary depending on such factors as the state of the autoimmune condition being treated, the overall health of the patient, method of administration, the severity of side-effects, and the like. Generally, IL-4R is administered as a pharmaceutical composition comprising an effective amount of IL-4R and a pharmaceutical carrier. A pharmaceutical carrier can be any compatible, non-toxic substance suitable for delivering the compositions of the invention to a patient. When administered parenterally, the IL-4R is formulated in a unit dosage injectable form (e.g., solution, suspension, or emulsion) in association with a pharmaceutical carrier. Such carriers are inherently nontherapeutic and nontoxic. Examples of such carriers are normal saline, Ringer's solution, dextrose solution, and Hank's solution. Nonaqueous carriers such as fixed oils and ethyl oleate may also be used. A preferred carrier is 5% dextrose/saline. The carrier may contain minor amounts of additives such as substances that enhance isotonicity and chemical stability, e.g., buffers and preservatives. The IL-4R is preferably formulated in purified form substantially free of aggregates and other proteins at a concentration in the range of about 5 to 20 $\mu$g/ml. Preferably, IL-4R is administered by continuous infusion so that an amount in the range of about 50-800 $\mu$g is delivered per day (i.e. about 1-16 $\mu$g/kg/day). The daily infusion rate may be varied based on monitoring of side effects and IL-4 and/or IgE levels.

Preferably, IL-4R is purified from culture supernatants of mammalian cells transiently transfected or stably transformed by an expression vector carrying an IL-4R gene. Most preferably, IL-4R is purified from culture supernatants of COS 7 cells transiently transfected by the pcD expression vector. Transfection of COS 7 cells with pcD proceeds as follows: One day prior to transfection, approximately 106 COS 7 monkey cells are seeded onto individual 100 mm plates in Dulbecco's modified Eagle medium (DME) containing 10% fetal calf serum and 2 mM glutamine. To perform the transfection, the medium is aspirated from each plate, washed with DME containing 50 mM Tris.HCl pH 7.4, and replaced with 4 ml of DME containing 50 mM Tris.HCl pH 7.4, 400 $\mu$g/ml DEAE-Dextran and 5 $\mu$g of plasmid DNA. The plates are incubated for four hours at 37° C, and then the DNA-containing medium is removed, and the plates are washed once with 5 ml of PBS, and then DME containing 10% fetal calf serum, 2 mM glutamine, penicillin and streptomycin is added. The cells are then incubated for 72 hrs at 37° C, after which the growth medium is collected for purification of IL-4R. Alternatively, transfection can be accomplished by electroporation as described in the Examples. Plasmid DNA for the transfections is obtained by growing pcD(SR$\alpha$) containing the IL-4R cDNA insert in E. coli MC1061, described by Casadaban and Cohen, J. Mol. Biol. , Vol. 138, pgs. 179-207 (1980), or like organism. The plasmid DNA is isolated from the cultures by standard techniques, e.g. Maniatis et al., Molecular Cloning: A Laboratory Manual (Cold Spring Harbor Laboratory, New York, 1982).

EXAMPLES

Example I. Construction of cDNA library from MC/9 cells and isolation of clone 19

A cDNA library was constructed in the pCEV4 vector from mRNA extracted from MC/9 cells, disclosed in U.S. patent 4,559,310. pCEV4 pcD and its derivative, pcD(SRα), are described by Okayama and Berg, Mol. Cell. Biol. , Vol. 2: 161-170 (1982) and Vol. 3: 280-289 (1983), and by Takebe et al., Mol. Cell. Biol. , Vol. 8, pgs. 466-472 (1988), respectively. The polyoma virus origin (the BglI-BclI fragment) was inserted into the Nde I site of pcD(SRα) for replication in mouse cells, and a polylinker sequence containing two Bst XI sites was inserted in the Pst I site of pcD(SRα). Size-selected double stranded cDNAS (>1.5 kilobases) having Bst XI adaptors were inserted into Bst XI-digested pCEV4, amplified in E. coli , and used to transfect COS 7 monkey cells by protoplast fusion (e.g. Sandri-Goldin et al., Mol. Cell. Biol. , Vol. 1, pgs. 743-752 (1981)). Populations of COS cells enriched for those expressing IL-4R on their surfaces were obtained by a modified panning technique based on that described by Seed (e.g. Proc. Natl. Acad. Sci. USA , Vol. 84, pgs. 3365-3369 (1987), or Proc. Natl. Acad. Sci. USA , Vol. 84, pgs 8573-8577 (1987)). Briefly, the modification consisted of the following steps: (1) biotin was attached to lysine residues of mouse IL-4, (2) prior to panning, the biotinylated IL-4 was incubated with the transfected COS cells so that the biotinylated IL-4 could bind to IL-4Rs expressed on the COS cells, and (3) the bound biotinylated IL-4 was covalently cross linked to the receptors by bis(2-(succinimidooxycarbonyloxy)ethyl)sulfone (Pierce Chemical Company, Rockford, IL). After these initial three steps the standard panning protocol was employed using microtiter plates coated with an anti-biotin antibody to selectively bind COS cells expressing IL-4Rs. Panning resulted in the isolation of clone 19 carrying the cDNA whose sequence is listed in Formula I.

Example II. Inhibition of IL-4 binding to T cells bv soluble IL-4R

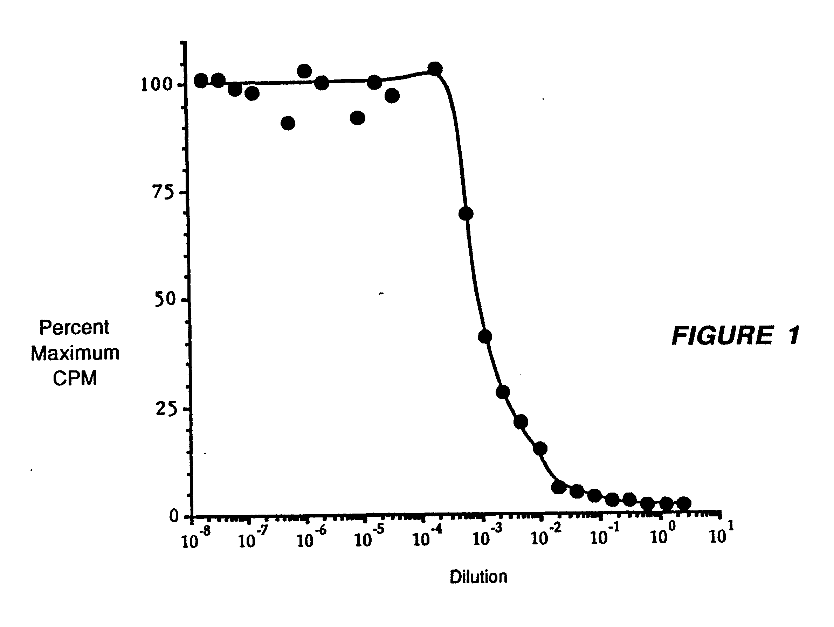

A cDNA encoding a soluble IL-4R was constructed from the IL-4R cDNA isolated in Example I by inserting linker CTAGACTAGTCTAG into a unique Bst EII restriction endonuclease site of clone 19 that was adjacent to and just 5´- of the transmembrane encoding domain. The resulting soluble IL-4R consisted of the extracellular domain of the receptor encoded by clone 19. The modified cDNA encoding the soluble IL-4R was transfected into COS 7 cells and the culture supernatants were assayed for inhibition of $^{125}$I-labeled IL-4 binding to the IL-4Rs of the mouse T cell line, HT-2; and for the inhibition of IL-4-induced growth of HT-2. The results are illustrated in Figures 1 and 2, respectively. In the growth inhibition assay the concentration of IL-4 was 100 picomolar.

Example III. Use of clone 19 to screen for a human IL-4R cDNA

Messenger RNA was prepared from the human Burkitt lymphoma cell line, BL41, and the human multi-factor dependent myeloid cell line, TF1, by the guanidium thiocyanate method. RNA was converted to cDNA, and Bst XI adaptors were attached to the cDNA. cDNAS larger than 2.0 kilobases were isolated by electrophoresis on a 0.8% agarose gel. The cDNA library of BL41 was constructed by inserting the size-selected cDNA into the Bst XI sites of the pCEV4 vector, a derivative of the pcD(SRα) expression vector described by Itoh et al., Science , Vol. 247, pg. 324 (1990). 1.2 x 10⁶ independent clones were obtained. A λgt11 phage cDNA library of TF1 was constructed by using the oligo(dT) primer containing the Not I restriction site and the Eco RI adaptor according to the protocol provided by Promega Corp. (Madison, WI).

The BL41 cDNA library was screened by colony hybridization using the mouse IL-4R cDNA as a probe. Colonies were transferred to nylon membrane filters (Schleicher & Schuell), and hybridization was performed at 42° C in 6 x SSPE (1 x SSPE contains 17.4 g/l NaCl, 2.76 g/l $NaH_2PO_4.H_2O$, 7.4 g/l EDTA, pH 7.4) containing 20% formamide, 5X Denhardt's solution, and 100 mg/ml E. coli tRNA. Filters were washed with 4 x SSPE solution at 50° C and exposed to X-ray film. Partial nucleotide sequence analysis showed that the longest cDNA clone from the BL41 library (4A2) had approximately 70 percent homology with the mouse IL-4R, but lacked about 700 bases from the 5´-end. 4A2 was then used as a probe to screen the TFI cDNA library under high stringency conditions (hybridization in 50% formamide at 42° C, and wash in 0.2 x SSPE at 60° C, otherwise the same as above). A clone (TFI-2.2) was obtained containing a 3.6 kilobase cDNA insert.

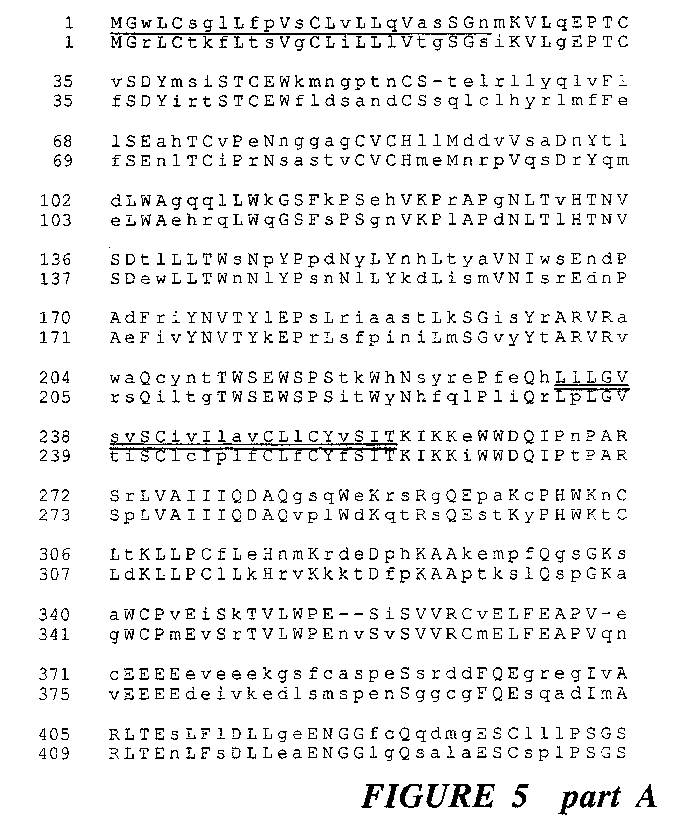

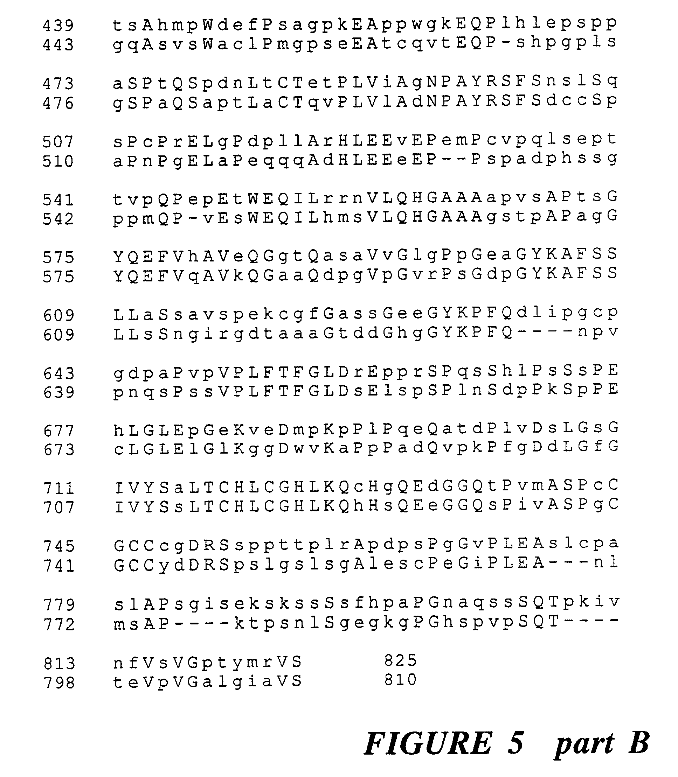

The nucleotide sequence of the 3.6 kilobase insert of the human IL-4R cDNA revealed a single long open reading frame encoding 825 amino acid residues including a putative signal sequence of 25 amino acid residues (indicated by underlining in Figure 5), an extracellular domain of 207 amino acid residues, a transmembrane domain (indicated by double underlining in Figure 5), and a cytoplasmic domain of 569 amino acid residues.

This soluble form of the human interleukin-4 receptor consists of an extracellular domain of the human interleukin-4 receptor. The extracellular domain typically consists of 200-220 N-terminal amino acids of the human interleukin-4 receptor. The 207-unit amino acid sequence prepared in the foregoing Example has the following amino acid sequence:

```
MKVLQEPTCV    SDYMSISTCE    WKMNGPTNCS    TELRLLYQLV
FLLSEAHTCV    PENNGGAGCV    CHLLMDDVVS    ADNYTLDLWA
GQQLLWKGSF    KPSEHVKPRA    PGNLTVHTNV    SDTLLLTWSN
PYPPDNYLYN    HLTYAVNIWS    ENDPADFRIY    NVTYLEPSLR
IAASTLKSGI    SYRARVRAWA    QCYNTTWSEW    SPSTKWHNSY
REPFEQH;
```

where the amino acids are designated by their single-letter abbreviations, namely

| A | Ala | Alanine | M | Met | Methionine |
|---|-----|---------|---|-----|------------|
| C | Cys | Cysteine | N | Asn | Asparagine |
| D | Asp | Aspartic acid | P | Pro | Proline |
| E | Glu | Glutamic acid | Q | Gln | Glutamine |
| F | Phe | Phenylalanine | R | Arg | Arginine |
| G | Gly | Glycine | S | Ser | Serine |
| H | His | Histidine | T | Thr | Threonine |
| I | Ile | Isoleucine | V | Val | Valine |
| K | Lys | Lysine | W | Trp | Tryptophane |
| L | Leu | Leucine | Y | Tyr | Tyrosine. |

Example IV. Binding properties of the human IL-4R

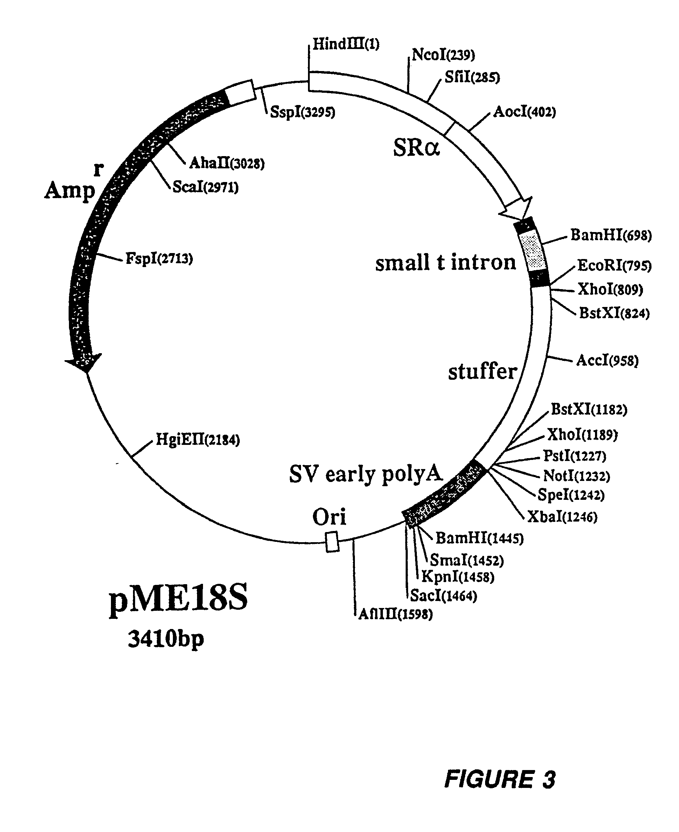

The full length human IL-4R cDNA was inserted into the mammalian expression vector, pME18S (Figure 3), and was transfected into COS 7 cells. pME18S-hIL-4R is deposited with the American Type Culture Collection (Rockville, MD) under accession number 68263. Binding assays were carried out as follows on the transfected COS 7 cells and the TF1 cells. COS 7 cells were detached from the plates by a short incubation in PBS containing 0.5 mM EDTA. Exponentially growing TF1 cells were harvested by centrifugation. Cells were washed and resuspended in RPMI 1640 containing 20 mM HEPES, and 0.5% BSA. Cells (7 x $10^4$ COS 7 cells or 1.2 x $10^6$ TFI cells) were incubated with 5-250 pM of $^{125}$I-IL-4 in the presence (solid squares or circles) and absence (empty squares or circles) of 10 nM nonradioactive IL-4 at 4°C for 3 hours. To prevent cell loss during washing, $10^6$ mouse MC/9 cells, which do not bind human IL-4, were added to each sample at the end of the incubation. Cell-bound radioactivity was separated by centrifugation as described by Galizzi et al., J. Biol. Chem. , Vol. 264, pg. 6984 (1989), which reference is incorporated by reference. The affinity of IL-4 binding to its receptor and the number of IL-4 receptors per cell were estimated by Scatchard plot analysis of equilibrium binding using the LIGAND program disclosed by Munson, Meth. Enzymol. , Vol. 92, pgs. 543-576 (1983).

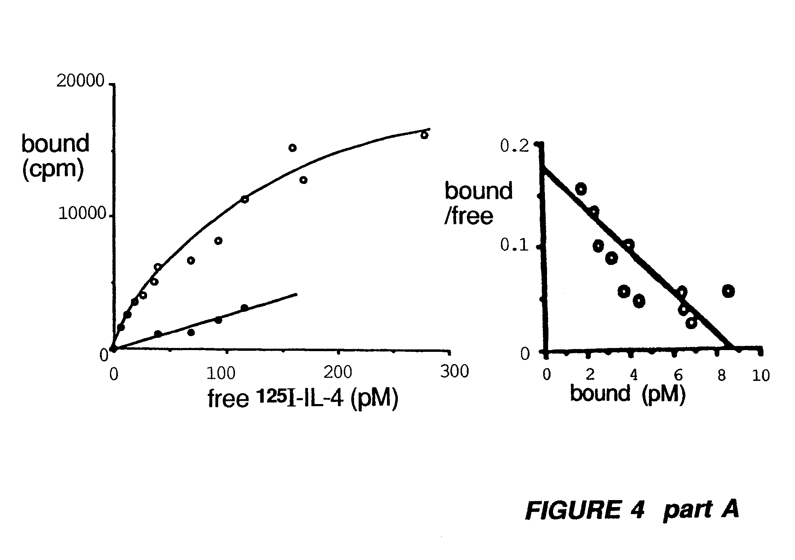

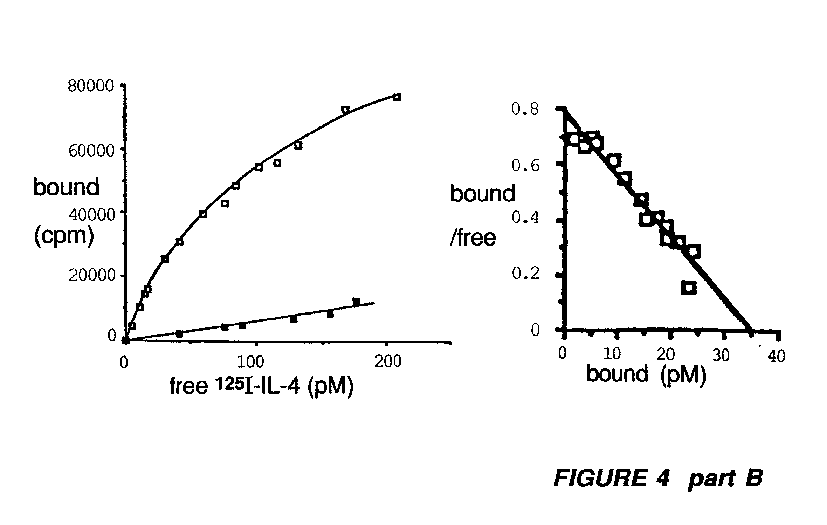

Equilibrium binding of $^{125}$I-human IL-4 to the transfected COS 7 cells and to the TF1 cells showed a single class of high affinity binding sites in both cells (Figure 4). Scatchard analysis indicated that COS 7 cells expressed 140,000 sites/cell with a binding constant, $K_d$, of 80-100 pM, while TF1 cells expressed about 1000 receptors/cell with a $K_d$ of 40-60 pM (Figure 4).

The descriptions of the foregoing embodiments of the invention have been presented for purpose of illustration and description. They are not intended to be exhaustive or to limit the invention to the precise forms disclosed, and obviously many modifications and variations are possible in light of the above teaching. The embodiments were chosen and described in order to best explain the principles of the invention to thereby enable others skilled in the art to best utilize the invention in various embodiments and with various modifications as are suited to the particular use contemplated. It is intended that the scope of the invention be defined by the claims appended hereto.

Applicants deposited E. coli MC1061 carrying pME18S-hIL-4R with the American Type Culture Collection, Rockville, MD, USA (ATCC), under accession number 68263, on March 20th 1990. This deposit was made under conditions as provided under ATCC's agreement for Culture Deposit for Patent Purposes, which assures that the deposit will be made available to the US Commissioner of Patents and Trademarks pursuant to 35 USC 122 and 37 CFR 1.14, and will be made available to the public upon issue of a U.S. patent, which requires that the deposit be maintained. Availability of the deposited strain is not to be construed as a license to practise the invention in contravention of the rights granted under the authority of any government in accordance with its patent laws.

The Deposits have been modified to comply with the requirements of the Budapest Treaty on the Deposit of Microorganisms.

## Claims

1. A nucleic acid encoding a mammalian interleukin-4 receptor.
2. The nucleic acid of claim 1 wherein said mammalian interleukin-4 receptor is a human interleukin-4 receptor.
3. The nucleic acid of claim 2 encoding a soluble form of said human interleukin-4 receptor.
4. The nucleic acid of claim 3 wherein said soluble form of said human interleukin-4 receptor consists of an extracellular domain of said human interleukin-4 receptor.
5. The nucleic acid of claim 4 wherein said extracellular domain consists of the 200-220 N-terminal amino acids of said human interleukin-4 receptor.
6. The nucleic acid of claim 2 which is effectively homologous to the nucleotide sequence of Formula I herein.
7. A soluble human interleukin-4 receptor consisting of the extracellular domain of the human interleukin-4 receptor.
8. The soluble human interleukin-4 receptor of claim 7 having the following amino acid sequence:

| | | | |
|---|---|---|---|
| MKVLQEPTCV | SDYMSISTCE | WKMNGPTNCS | TELRLLYQLV |
| FLLSEAHTCV | PENNGGAGCV | CHLLMDDVVS | ADNYTLDLWA |
| GQQLLWKGSF | KPSEHVKPRA | PGNLTVHTNV | SDTLLLTWSN |
| PYPPDNYLYN | HLTYAVNIWS | ENDPADFRIY | NVTYLEPSLR |
| IAASTLKSGI | SYRARVRAWA | QCYNTTWSEW | SPSTKWHNSY |
| REPFEQH. | | | |

9. Plasmid pME18S-hIL-4R deposited at the ATTC under accession no. 68263.

FIGURE 1

FIGURE 2

O.D. 570/650

INVERSE DILUTION

Constant mIL-4
Soluble IL-4R

0.08
0.06
0.04
0.02
0.00

$10^0$ $10^1$ $10^2$ $10^3$ $10^4$

**FIGURE 3**

FIGURE 4   part A

*FIGURE 4  part B*

```
  1    MGwLCsglLfpVsCLvLLqVasSGnmKVLqEPTC
  1    MGrLCtkfLtsVgCLiLLlVtgSGsiKVLgEPTC

 35    vSDYmsiSTCEWkmngptnCS-telrllyqlvFl
 35    fSDYirtSTCEWfldsandCSsqlclhyrlmfFe

 68    lSEahTCvPeNnggagCVCHllMddvVsaDnYtl
 69    fSEnlTCiPrNsastvCVCHmeMnrpVqsDrYqm

102    dLWAgqqlLWkGSFkPSehVKPrAPgNLTvHTNV
103    eLWAehrqLWqGSFsPSgnVKPlAPdNLTlHTNV

136    SDtlLLTWsNpYPpdNyLYnhLtyaVNIwsEndP
137    SDewLLTWnNlYPsnNlLYkdLismVNIsrEdnP

170    AdFriYNVTYlEPsLriaastLkSGisYrARVRa
171    AeFivYNVTYkEPrLsfpiniLmSGvyYtARVRv

204    waQcyntTWSEWSPStkWhNsyrePfeQhLlLGV
205    rsQiltgTWSEWSPitWyNhfqlPliQrLpLGV

238    svSCivIlavCLlCYvSITKIKKeWWDQIPnPAR
239    tiSClcIplfCLfCYfSITKIKKiWWDQIPtPAR

272    SrLVAIIIQDAQgsqWeKrsRgQEpaKcPHWKnC
273    SpLVAIIIQDAQplWdKqtRsQEstKyPHWKtC

306    LtKLLPCfLeHnmKrdeDphKAAkempfQgsGKs
307    LdKLLPClLkHrvKkktDfpKAAptkslQspGKa

340    aWCPvEiSkTVLWPE--SiSVVRCvELFEAPV-e
341    gWCPmEvSrTVLWPEnvSvSVVRCmELFEAPVqn

371    cEEEEeveeekgsfcaspeSsrddFQEgregIvA
375    vEEEEdeivkedlsmspenSggcgFQEsqadImA

405    RLTEsLFlDLLgeENGGfcQqdmgESClllPSGS
409    RLTEnLFsDLLeaENGGlgQsalaESCsplPSGS
```

*FIGURE 5  part A*

17

```
439   t s A h m p W d e f P s a g p k E A p p w g k E Q P l h l e p s p p
443   g q A s v s W a c l P m g p s e E A t c q v t E Q P - s h p g p l s

473   a S P t Q S p d n L t C T e t P L V i A g N P A Y R S F S n s l S q
476   g S P a Q S a p t L a C T q v P L V l A d N P A Y R S F S d c c S p

507   s P c P r E L g P d p l l A r H L E E v E P e m P c v p q l s e p t
510   a P n P g E L a P e q q q A d H L E E e E P - - P s p a d p h s s g

541   t v p Q P e p E t W E Q I L r r n V L Q H G A A A a p v s A P t s G
542   p p m Q P - v E s W E Q I L h m s V L Q H G A A A g s t p A P a g G

575   Y Q E F V h A V e Q G g t Q a s a V v G l g P p G e a G Y K A F S S
575   Y Q E F V q A V k Q G a a Q d p g V p G v r P s G d p G Y K A F S S

609   L L a S s a v s p e k c g f G a s s G e e G Y K P F Q d l i p g c p
609   L L s S n g i r g d t a a a G t d d G h g G Y K P F Q - - - - n p v

643   g d p a P v p V P L F T F G L D r E p p r S P q s S h l P s S s P E
639   p n q s P s s V P L F T F G L D s E l s p S P l n S d p P k S p P E

677   h L G L E p G e K v e D m p K p P l P q e Q a t d P l v D s L G s G
673   c L G L E l G l K g g D w v K a P p P a d Q v p k P f g D d L G f G

711   I V Y S a L T C H L C G H L K Q c H g Q E d G G Q t P v m A S P c C
707   I V Y S s L T C H L C G H L K Q h H s Q E e G G Q s P i v A S P g C

745   G C C c g D R S s p p t t p l r A p d p s P g G v P L E A s l c p a
741   G C C y d D R S p s l g s l s g A l e s c P e G i P L E A - - - n l

779   s l A P s g i s e k s k s s S s f h p a P G n a q s s S Q T p k i v
772   m s A P - - - - k t p s n l S g e g k g P G h s p v p S Q T - - - -

813   n f V s V G p t y m r V S        825
798   t e V p V G a l g i a V S        810
```

# FIGURE 5   part B

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 111, no. 25, 18th December 1989, page 564, abstract no. 230163v, Columbus, Ohio, US; N. HARADA: "IL-4 receptor", & MED. IMMUNOL. 1989, 18(2), 209-13 * The whole document * | 1-8 | C 12 N 15/12 C 07 K 13/00 C 12 P 21/02 |
| P,X | WO-A-9 005 183 (IMMUNEX CORP.) * Examples; figures 4A-4C * | 1-8 | |
| A | JOURNAL OF EXPERIMENTAL MED., vol. 166, August 1987, pages 476-488, New York, US; L.S. PARK et al.: "Characterization of the human B cell stimulatory factor 1 receptor" * The whole document * | 1 | |
| A | IMMUNOLOGY, vol. 65, 1988, pages 617-622; M. LARCHE et al.: "Functional evidence for a monoclonal antibody that binds to the human IL-4 receptor" * The whole document * | 1 | |
| | | | TECHNICAL FIELDS SEARCHED (Int. Cl.5) C 12 N C 12 P |

The present search report has been drawn up for all claims

| Place of search | Date of completion of search | Examiner |
|---|---|---|
| The Hague | 08 January 91 | LE CORNEC N.D.R. |